Europäisches Patentamt

European Patent Office

Office européen des brevets

(19)

(11) Veröffentlichungsnummer: **0 219 804 B1**

(12) # EUROPÄISCHE PATENTSCHRIFT

(45) Veröffentlichungstag der Patentschrift: **23.12.92**

(21) Anmeldenummer: **86114236.2**

(22) Anmeldetag: **15.10.86**

Die Akte enthält technische Angaben, die nach dem Eingang der Anmeldung eingereicht wurden und die nicht in dieser Patentschrift enthalten sind.

(51) Int. Cl.5: **C01B 33/34**, C01B 35/10, C01B 33/20, B01J 29/28, B01J 29/04, C07C 1/20

(54) **Verfahren zur Herstellung grosser Zeolithkristalle.**

(30) Priorität: **22.10.85 DE 3537459**

(43) Veröffentlichungstag der Anmeldung:
**29.04.87 Patentblatt 87/18**

(45) Bekanntmachung des Hinweises auf die Patenterteilung:
**23.12.92 Patentblatt 92/52**

(84) Benannte Vertragsstaaten:
**BE DE FR GB NL**

(56) Entgegenhaltungen:
**EP-A- 0 091 508**
**EP-B- 0 041 621**
**GB-A- 2 127 036**

**"ULLMANNS ENCYCLOPÄDIE DER TECHNI-SCHEN CHEMIE", Band 21, 4. Auflage, 1982, Verlag Chemie, Weinheim, Seiten 462-465**

**RÖMPP CHEMIE LEXIKON, 9. Ausgabe, Seite 65**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Schlimper, Hans-Ulrich, Dr.**
**Im Erlich 74**
**W-6720 Speyer(DE)**

EP 0 219 804 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 219 804 B1

**Beschreibung**

Die Erfindung betrifft ein Verfahren zur Herstellung von ebenmäßig ausgebildeten Kristallen von Zeolithen des Pentasiltyps aus hochdispersem siliciumdioxid, das durch Verbrennung einer Siliciumchlorverbindung erhalten wird, und einer Verbindung dreiwertiger Elemente in aminhaltiger Lösung.

Aus der EP-A-41 621 ist ein Verfahren zur Herstellung von Zeolithen vom Strukturtyp ZSM-5 durch hydrothermale Kristallisation bei Temperaturen von 100 bis 200°C aus Mischungen von $SiO_2$ und $Al(OH)_3$ in Abwesenheit von Alkali in wäßrigen Lösungen von Aminen bekannt, welches dadurch gekennzeichnet ist, daß man die Kristallisation in Gegenwart von Triaminen, Tetraminen und/oder höheren Aminen durchführt.

Kristalline Aluminosilikate, wie Zeolithe, natürlicher als auch synthetischer Herkunft, haben sich als katalytisch wirksame Stoffe für verschiedene Arten von Kohlenwasserstoffumwandlung, z.B. beim Cracken und Isomerisieren von Kohlenwasserstoffen, bei der Aromatenalkylierung, bei der Methanolumwandlung zu Olefinen und Benzingemischen, erwiesen. Aber auch als Ionenaustauscher und Molekularsiebe finden Zeolithe technische Verwendung.

Zeolithe besitzen eine hochgeordnete Struktur mit einem starren dreidimensionalen Netzwerk von $SiO_4$ - und $AlO_4$-Tetraedern, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall z.B. eines Alkali- oder Wasserstoffions ausgeglichen. Ein Kationenaustausch ist möglich. Die Raum zwischen den Tetraedern sind vor der Dehydratation durch Trocknen bzw. Calcinieren von Wassermolekeln besetzt.

In den letzten Jahren haben kristalline Aluminosilikate mit einem hohen $SiO_2/Al_2O_3$-Verhältnis $\geq 11$ zunehmendes Interesse gewonnen. Derartige Zeolithe zeichnen sich durch hohe thermische Stabilität und außerordentlich hohe Acidität aus. Die Synthese von Zeolithen mit Pentasil-Struktur erfolgt in bekannter Weise aus einer Silicium- und einer Aluminiumkomponente in Gegenwart von voluminösen quaternären organischen Ammoniumverbindungen und Alkaliverbindungen als Mineralisierungsmittel. Ein derartiges Verfahren ist z.B. in US-A-3 702 886 beschrieben. Die großen Ammoniumionen sollen als Muster für die gewünschte Zeolithstruktur wirken und ermöglichen die Synthese von kristallinen Aluminiumsilikaten mit $SiO_2/Al_2O_3$-Verhältnissen von über 100, z.B. bis 40 000. In Gegenwart von derartigen Zeolithen kann man z.B. die Umwandlung von Methanol unter C-Verknüpfung in Olefine ausführen (US-A-3 911 041) oder die Alkylierung des Benzols mit Ethylen zu Ethylbenzol katalysieren (US-A-3 751 504).

Dabei erhält man im allgemeinen kristalline oder gemischt kristalline Zeolithe mit Kristallen verschiedener Größe z.B. 0,5 bis 1,0 $\mu$m und verwachsener Form die teilweise oder ganz agglomerieren. Für verschiedene Anwendungen der Zeolithe insbesondere vom Pentasiltyp bei der Katalyse von Umwandlungen organischer Verbindungen ist es aber wünschenswert Zeolithe einzusetzen, die sich aus großen ebenmäßig ausgebildeten Kristallen zusammensetzen.

Demgemäß wurde ein Verfahren zur Herstellung großer ebenmäßig ausgebildeter Kristalle von Zeoli-then des Pentasiltyps aus $SiO_2$,mit einem $SiO_2$-Gehalt größer oder gleich 99,8 % und einer Verbindung eines oder mehrerer dreiwertiger Elemente wie Al, B, Fe, Ga, Cr in aminhaltigen Lösungen, das durch Verbrennung einer Siliciumchlorid-Verbindung hergestellt wurde, gefunden, welches dadurch gekennzeichnet ist, daß man als $SiO_2$-haltigen Ausgangsstoff hochdisperses $SiO_2$ mit einer BET-Oberfläche von 200 ± 25 m$^2$/g und mit 10 bis 50 ppm Cl und 5 bis 80 ppm F verwendet.

Als hochdisperses $SiO_2$ verwendet man ein $SiO_2$ das durch Verbrennung einer Siliziumchlorid-Verbindung hergestellt wird. Der $SiO_2$-Ausgangsstoff soll Vorteilhaft eine Oberfläche (BET) von 200 ± 25 m$^2$/g und eine Teilchengröße von 0,012 $\mu$m haben, einen $SiO_2$-Gehalt größer oder gleich 99.8 % mit 10 bis 50 ppm Cl und 5 bis 80 ppm F, besonders geeignet ist das Handelsprodukt CAB-O-SIL M5®.

Pentasilzeolithsynthesen kann man in Polyamin-Lösungen wie Hexamethylendiamin, Propandiamin auch ohne Zusatz von Alkalionen durchführen. Man kann aber auch ätherische und alkoholische Lösungen bzw. Mischungen derselben verwenden. Mit dem Verfahren kann man auch vorteilhaft isotaktische Zeolithe gewinnen.

Es war überraschend, daß man durch Einsatz von hochdispersem $SiO_2$, das durch Verbrennung von Silicium-chlorverbindungen erhalten wurde, aus aminhaltiger Lösung große wohlgeformte, nicht zu Agglomeraten verwachsene Einzelkristalle erhalten kann. Da die Größe und das Vorliegen von isolierten Primärkristallen einen Einfluß auf das Diffusionsverhalten von Reaktanten, die Verweilzeit im Zeolithkanal und damit Einfluß auf die katalytische Wirksamkeit hat, sind Zeolithsynthesen, die gezielt zu Zeolithen einheitlicher Kristallgrößen führen, wünschenswert.

Durch das erfindungsgemäße Verfahren wird ein neuer Weg eröffnet, die Kristallgröße zu beeinflussen.

Als Verbindungen dreiwertiger Elemente sind z.B. geeignet die Oxide, Sulfate und Nitrate von Al, B, Fe, Ga und Cr.

2

Eine bevorzugte Ausführungsform der erfindungsgemäßen Zeolithsynthese besteht darin, daß man eine Reaktionsmischung aus hochdispersem Siliciumdioxid, das durch Verbrennung von $SiCl_4$ bei erhöhter Temperatur großtechnisch hergestellt wird, beispielsweise mit $Al_2(SO_4)_3$ in aminhaltiger Lösung z.B. in 50 %iger wäßriger Propandiamin-Lösung durch hydrothermale Kristallisation 1 bis 5 Tage bei 140 bis 200°C unter autogenem Druck in einem Rührautoklaven umsetzt. Die hierbei erhaltene Produkte enthalten Amine und Wasser in den intrakristallinen Poren. Durch Trocknung bei 100 bis 160°C und durch Calcination bis 450°C bis 570°C kann man das Wasser und das Amin entfernen und man erhält so die Zeolithe in der katalytisch aktiven, aciden H-Form.

Die Synthese wird im allgemeinen bei 150 bis 170°C, vorteilhaft bei 160 bis 165°C und bei dem autogenen Druck der Lösung unter Rühren im Rührautoklaven durchgeführt.

Die erfindungsgemäß hergestellten Zeolithe kann man allgemein für die Umwandlung organischer Verbindungen, z.B. für die Herstellung von niederen Olefinen und Aromaten aus Methanol und die Alkylierung von Aromaten verwenden.

Beispiel 1

7 kg hochdisperses Siliciumdioxid (CAB-O-SIL M5®), das durch Verbrennung von $SiCl_4$ erhalten wird, wird in 100 kg einer 50 %igen wäßrigen 1,3-Diaminopropan-Lösung in einem Rührautoklaven vorgelegt. 1,96 kg $Al_2(SO_4)_3 \cdot 18H_2O$ werden in 7 kg $H_2O$ gelöst und zur obigen Mischung zugegeben. Bei 165°C wird unter autogenem Druck 5 Tage gerührt. Nach Abfiltrieren und Auswaschen des Zeolithen wird bei 140°C getrocknet und bei 500°C calciniert.

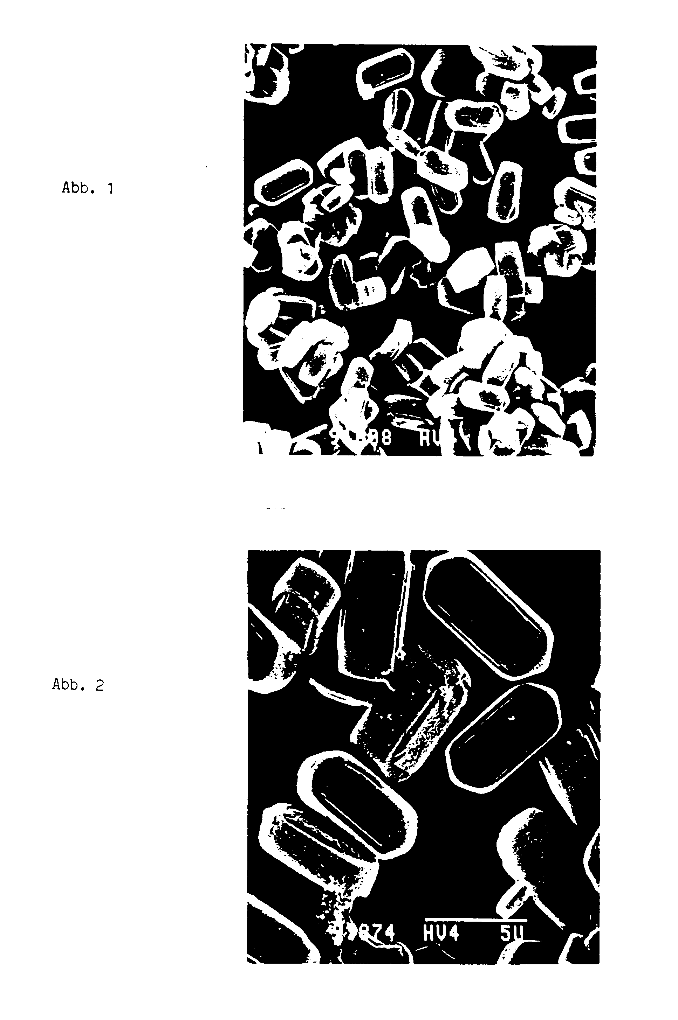

Dieser Aluminosilikatzeolith des Pentasiltyps enthält 93,3 Gew.% $SiO_2$, 3,5 Gew.% $Al_2O_3$ und Restwasser. Der Kristallhabitus wird in der rasterelektronenmikroskopischen Aufnahme Abb. 1 und 2 gezeigt. Diese beiden Abbildungen geben verschiedene photographische Vergrößerungen wieder.

Der oben beschriebene Aluminosilikatzeolith wird mit Boehmit im Gewichtsverhältnis 60:40 zu 2 mm Strängen verformt. Die Stränge werden bei 110°C getrocknet und bei 500°C/16 h calciniert.

An diesem Katalysator wird 25 %ige wäßrige Methanollösung zu Kohlenwasserstoffen umgesetzt. Die Reaktion in der Gasphase wird unter isothermen Bedingungen in einem Rohrreaktor (Wendel, 0,6 cm Innendurchmesser, 90 cm Länge) durchgeführt. Die Versuchsergebnisse sind in der Tabelle zusammengefaßt.

Beispiel 2

An dem nach Beispiel 1 hergestellten Katalysator wird in der dort beschriebenen Apparatur ein Gemisch aus Benzol/Ethylen (3:1 molar) bei 400°C und WHSV = 7,8 $h^{-1}$ umgesetzt. Der Umsatz an Benzol beträgt 26,2 % und die Selektivität an Ethyl- und Diethylbenzol, bezogen auf Benzol, 99,2 %.

**Tabelle**

| | 300°C | 400°C | 550°C |
|---|---|---|---|
| Temperatur | | | |
| WHSV[1] | 2,7 $h^{-1}$ | 2,7 $h^{-1}$ | 2,7 $h^{-1}$ |
| **Ausbeute %** | | | |
| $C_2H_4$ | 24,3 | 17,8 | 29,8 |
| $C_3H_6$ | 11,3 | 15,7 | 19,7 |
| $C_4H_8$ und $C_4H_{10}$ | 12,4 | 15,9 | 9,5 |
| $CH_4$ | 0,2 | 1,8 | 7,6 |
| $C_2H_6$ | 0,2 | 0,2 | 1,1 |
| $C_3H_8$ | 6,8 | 3,5 | 6,7 |
| $C_5^{+}$[2] | 44,0 | 45,1 | 25,6 |

1) WHSV = g $CH_3OH$/g Katalysator x h

2) aliphatische und aromatische Verbindungen

**Patentansprüche**

1. Verfahren zur Herstellung großer ebenmäßig ausgebildeter Kristalle von Zeolithen des Pentasiltyps aus $SiO_2$, mit einem $SiO_2$-Gehalt größer oder gleich 99,8 % und einer Verbindung eines oder mehrerer dreiwertiger Elemente wie Al, B, Fe, Ca, Cr in aminhaltigen Lösungen, das durch Verbrennung einer Siliciumchlorid-Verbindung hergestellt wurde, dadurch gekennzeichnet, daß man als $SiO_2$-haltigen Ausgangsstoff hochdisperses $SiO_2$ mit einer BET-Oberflache von $200 \pm 25$ $m^2$/g und mit 10 bis 50 ppm Cl und 5 bis 80 ppm F verwendet.

2. Verwendung der nach Anspruch 1 hergestellten Zeolithkristalle für die Umwandlung organischer Verbindungen.

3. Verwendung der nach Anspruch 1 hergestellten Zeolithkristalle für die Umwandlung von Methanol zu Kohlenwasserstoffen die niedere Olefine und Aromaten enthalten.

**Claims**

1. A process for preparing large uniform crystals of zeolites of the pentasil type from $SiO_2$ having an $SiO_2$ content equal or greater than 99.8 % and prepared by burning a silicon chloride compound and from a compound of one or more trivalent elements such as Al, B, Fe, Ga, Cr in amine-containing solutions, which comprises as the $SiO_2$-containing starting material a finely divided $SiO_2$ having a BET surface area of $200 \pm 25$ $m^2$ and containing from 10 to 50 ppm of Cl and from 5 to 80 ppm of F.

2. The use of the zeolite crystals prepared as claimed in claim 1 for the interconversion of organic compounds.

3. The use of the zeolite crystals prepared as claimed in claim 1 for converting methanol into hydrocarbons that contain lower olefins and aromatics.

**Revendications**

1. Procédé de fabrication de gros cristaux de même forme de zéolithes du type pentasil à partir d'un $SiO_2$ qui a une teneur en $SiO_2$ supérieure ou égale à 99,8% et qui a été préparé par combustion d'un composé à base de chlorure de silicium et à partir d'un composé d'un ou de plusieurs éléments trivalents tels que Al, B, Fe, Ga, Cr, dans une solution contenant une amine, caractérisé en ce qu'on utilise, comme matière de départ contenant $SiO_2$, du $SiO_2$ fortement dispersé ayant une surface spécifique BET de $200 \pm 25$ $m^2$ et contenant de 10 à 50 ppm de Cl et de 5 à 80 ppm de F.

2. Utilisation des cristaux de zéolithe fabriqués selon la revendication 1 pour la transformation de composés organiques.

3. Utilisation des cristaux de zéolithe fabriqués selon la revendication 1 pour la transformation de méthanol en hydrocarbures qui contiennent des oléfines et des carbures aromatiques inférieurs.

Abb. 1

Abb. 2